# EUROPEAN PATENT APPLICATION

(11) **EP 4 628 102 A2**
(43) Date of publication of application: **08.10.2025**
(21) Application number: 25187260.2
(22) Date of filing: 08.11.2021
(51) Int. Cl.: A61K 47/14

(54) **STABLE TOPICAL NANOEMULSION COMPRISING MEGLUMINE ANTIMONIATE AND AMPHOTERICIN B FOR THE TREATMENT OF CUTANEOUS LEISHMANIASIS**

(30) Priority: 09.11.2020 BR 102020022824
(62) Divisional of application: 21887931.0
(71) Applicant: Fundação Oswaldo Cruz, 21045-900 Rio de Janeiro, RJ (BR)
(72) Inventor: Rabello, Ana, Lucia, Teles, 34009-580 Nova Lima- MG, Brasil (BR); Costa, Jorge, Carlos, Santos da, 22210-085 Rio de Janeiro- RJ, Brasil (BR); Queiroz, Dinalva, Brito de, 60760-000 Eusébio- CE, Brasil (BR); Teixeira, Eliane, de Morais, 30130-180 Belo Horizonte- MG, Brasil (BR)
(74) Representative: Weidner Stern Jeschke

(57) **Abstract**

The drugs available for the treatment of cutaneous leishmaniasis have unsatisfactory efficacy, frequent and serious adverse effects, and require long treatment regimens. Thus, the search for new treatment alternatives for cutaneous leishmaniasis is considered a priority by the World Health Organization. Parenteral administration of pentavalent antimonials for the treatment of all forms of leishmaniasis, including cutaneous leishmaniasis, has several limitations. The therapy is long, requires repeated doses, and adverse reactions are frequent. Topical treatment is an attractive alternative for cutaneous leishmaniasis, offering significant advantages over systemic therapy: fewer adverse effects, ease of administration, and lower costs. The present inventors aimed to provide a fixed-dose topical composition containing at least one antileishmanial compound, providing adequate absorption of the active ingredient. Another objective of the present invention is to provide a topical, fixed-dose formulation containing a combination of antileishmanial compounds that has sufficient efficacy and safety to be used in the treatment of cutaneous leishmaniasis.

## Description

### FIELD OF INVENTION

Tegumentary leishmaniasis is a neglected disease caused by species with dermal trophism of the protozoan *Leishmania* spp. that affects the skin (cutaneous leishmaniasis) and mucous membranes (mucosal leishmaniasis), with a chronic profile. The main control measures include appropriate diagnosis and treatment of the disease in order to reduce the suffering of patients, mainly due to unfavorable outcomes such as deformities and death.

In the search for therapeutic alternatives for cutaneous leishmaniasis, different topical formulations have been evaluated for over thirty years, due to their potential ease of administration and reduced adverse effects.

However, currently available drugs for cutaneous leishmaniasis still have unsatisfactory efficacy and frequent and serious adverse effects. Furthermore, most of the available treatment methods are still based on parenteral drug administration and require long periods of treatment. Thus, the search for new treatment alternatives for cutaneous leishmaniasis ensues.

### BACKGROUND OF THE INVENTION

Leishmaniasis is caused by protozoan parasites that are transmitted by the bite of sandflies that inject infectious metacyclic promastigotes under the skin of the vertebrate host. In vertebrate hosts, the leishmania survives at the site of infection, transforms into non-motile amastigote forms, and multiplies within the cells of the mononuclear phagocytic system. Eventually, depending on various factors, the parasites can spread to other sites on the skin or mucous membranes, especially in the upper respiratory tract. Therefore, after the infection, patients can develop symptoms that vary in severity, from skin lesions with significant impairment of quality of life to severe disfigurement.

Leishmaniasis mainly affects low-income people in Africa, Asia, and Latin America and is associated with malnutrition and a weakened immune system. Of the approximately 200 countries and territories that report cases to the World Health Organization (WHO), 97 are endemic for leishmaniasis. In 2014, over 90% of new cases of leishmaniasis (visceral and cutaneous) reported to the WHO occurred in Brazil, Ethiopia, India, Somalia, South Sudan, and Sudan (WHO, 2018). The WHO estimates that there are 600,000 to l million new cases of tegumentary leishmaniasis each year worldwide; about 95 percent of these cases occur in the Americas, the Mediterranean Basin, the Middle East, and Central Asia.

The leishmaniases are a group of different diseases that can compromise the skin, mucous membranes, and viscera. They are caused by more than 20 species of protozoa of the genus *Leishmania,* grouped into two subgenera: *Leishmania* and *Viannia. Leishmania* is a heterogeneous parasite with vertebrate and invertebrate hosts, such as the dipterous insects of the subfamily *Phlebotominae,* belonging to the genera *Phlebotomus* and *Lutzomyia.*

Cutaneous leishmaniasis (CL) results in the formation of lesions (mostly ulcerated) on the skin at the site of the sandfly bite. CL is mainly caused by *Leishmania (Leishmania) major, L. (L.) tropica, L. (L.) aethiopica, L. (Viannia) braziliensis, (L.) (V.) guyanensis L (V.) panamensis, L. (L.) amazonensis.* and *L. (L.) mexicana.* The disease is usually self-limited, but infection by New World species, for example, can spread to the lymph nodes and other sites on the skin or mucous membranes. Among the clinical presentations of tegumentary leishmaniasis, the most severe is caused by the most prevalent species in the Americas, *L. (V.) braziliensis,* which develops after healing of the initial skin lesions. The late development of metastatic lesions leads to partial or total destruction of the mucous membranes of the nose, oropharynx, and larynx, progressing to severe deformities or even death.

Regarding the treatment of cutaneous leishmaniasis, the Ministry of Health in Brazil recommends pentavalent antimonial as the first-choice drug. In the absence of satisfactory response, situations of toxicity, or formal contraindication, the use of liposomal amphotericin B, amphotericin B deoxycholate, and pentamidine isethionate is recommended (BRAZIL, 2017).

The drugs recommended for the treatment of cutaneous leishmaniasis have unsatisfactory efficacy, have frequent and serious adverse effects, and require long treatment regimens. Thus, the search for new treatment alternatives for cutaneous leishmaniasis is a priority highlighted by international organizations and the Brazilian Ministry of Health.

Topical treatment is an attractive alternative for the treatment of cutaneous leishmaniasis. Topical formulations offer significant advantages over systemic therapy: fewer adverse effects, ease of administration, and reduced cost.

### Currently used drugs

### Pentavalent Antimonials

Antimonial compounds, in the form of trivalent salts, were first used to treat cutaneous leishmaniasis in 1912 by Gaspar Vianna, shortly after the recognition in 1904 that protozoa of the genus *Leishmania* were the causative agents of the leishmaniases (BERMAN et al., 1988). There are two different formulations of pentavalent antimony (Sb+5) commercially available, meglumine antimoniate and sodium stibogluconate.

Meglumine antimoniate is the first-choice drug for the treatment of cutaneous leishmaniasis in Brazil. It is recommended for patients from all Brazilian regions, except patients with renal, hepatic, or cardiac comorbidity, pregnant women, and patients who are 50 years old or older. It is also not indicated for patients coming from the northern part of the country, where *L. (V.) guyanensis* occurs and does not respond well to treatment with meglumine antimoniate, so pentamidine isethionate is used. Meglumine antimoniate is used muscularly or intravenously at a dose of 15 to 20 mg/Kg/day for 20 to 30 consecutive days. It is considered a highly toxic drug with cumulative adverse effects; among several serious adverse effects, the most important are cardiac, hepatic, pancreatic, and/or renal alterations (BRAZIL, 2017). In addition, the use of this drug requires constant outpatient monitoring of the patient, which requires technical resources and trained professionals.

The limited use of meglumine antimoniate for certain patient groups and its high toxicity make it necessary to evaluate new therapeutic presentations or different protocol proposals.

### Amphotericin B

Amphotericin B is a polyene antibiotic produced by different *Streptomyces* species. Leishmanicidal activity was first demonstrated in the 1950s, and this drug started to be used for the treatment of leishmaniases (DONOVLICK et al., 1956; SAHA et al., 1968). It is formulated as a colloidal suspension of lyophilized sodium amphotericin B deoxycholate and commercialized in Brazil as Anforicin B^{®} (50 mg - Cristália/Brazil).

The Brazilian Ministry of Health indicates the use of amphotericin B deoxycholate as a second-choice drug for the treatment of cutaneous leishmaniasis, recommended in situations of toxicity or refractory to treatment with antimonial. The recommended dose is 0.5 to 1.0 mg/kg/day, with a total dose of 25 to 40 mg/kg, given intravenously over four to six hours and solubilized in a 5% glucose solution. Treatment requires hospitalization and is contraindicated for patients with renal failure. Among its adverse effects, impairment of kidney function is considered the most serious (BRAZIL, 2017a).

### Pentamidine Isethionate

Pentamidine, an aromatic diamidine with antimicrobial potential, was synthesized as a hypoglycemic drug and had its leishmanicidal activity demonstrated (ROBERT; BRIGGAMAN, 1977; BALAÑA-FOUCE et al., 1998).

It is presented as pentamidine isethionate (Di-B-Hydroxyethane Sulfonate) in an ampoule containing 300 mg of the salt. The Brazilian Ministry of Health recommends its use as the first-choice drug for the treatment of localized cutaneous leishmaniasis caused by *L. (V.) guyanensis* and as the second-choice drug for the other clinical forms of tegumentary leishmaniasis. Pentamidine is not recommended for patients with renal, hepatic, or cardiac comorbidity, diabetics, pregnant and lactating women, and children under one year of age. The treatment protocol ranges from three to ten doses of 3 to 4 mg/kg/day on alternate days and can be performed intramuscularly or intravenously (BRAZIL, 2017a).

### Miltefosine

Miltefosine (hexadecylphosphocholine) is an alkylphosphocholine, originally developed as an antineoplastic agent, that is being incorporated into topical formulations for the treatment of skin metastases from breast cancer. In the 1980s, its leishmanicidal activity was demonstrated against *Leishmania* species causing visceral leishmaniasis (HERRMANN et al., 1982; ACHTERBERG et al., 1987; CROFT et al., 1987). Subsequently, Escobar and collaborators (2002) demonstrated the efficacy of this drug in an *in vitro* study, not only *for L.* (*L.*) *donovani* but also for species causing the cutaneous form of the disease (*L.* (*L*.) *major, L. (L.) tropica, L. (L.) aethiopica, L. (L.) mexicana* and *L.* (*V.*) *panamensis*) (ESCOBAR et al., 2002). Old World patients with cutaneous leishmaniasis due to *L.*(*L.*) *major* had a 93% cure rate with miltefosine treatment (MOHEBALI et al., 2007). The limitation of miltefosine is its teratogenic potential, which limits its use in patients in the reproductive phase, and pregnancy testing is indicated before its use in these patients and family counseling during treatment. It is also a drug with a high frequency of moderate adverse effects, such as nausea, vomiting, and diarrhea.

### Pentavalent Intralesional Antimonial

The use of meglumine antimoniate, Glucantime^{®}, administered intralesionally, has recently been introduced in the recommendations of the Brazilian Ministry of Health as the first choice regimen for the treatment of localized cutaneous leishmaniasis with a single lesion up to 3 cm in diameter. The treatment protocol is one to three applications of approximately 5 mL per session, with the window between applications being 15 days (BRAZIL, 2017). This therapeutic approach is distinguished by the local application route, which reduces the toxicity of this treatment. Moreover, this procedure does not require investment in equipment or hospitalization of the patients.

### Paromomycin

Paromomycin is an antibiotic of the aminoglycoside class, first isolated in 1956, and is produced by *Streptomyces rimosus,* subspecies *paromomycinus* (SUNDAR; CHAKRAVARTY, 2008). It is commercialized in two presentations: Paramomycin^{®} (Gland Pharma Ltd., India) and Leshcutan^{®} (Teva Pharmceutical Industries Ltd., Israel).

Paromomycin has been evaluated for the treatment of cutaneous leishmaniasis, mainly in the Old World, and its topical application is considered ideal for its ease of administration and low frequency of adverse effects (BERMAN., 2005; CARNEIRO et al., 2012). The first studies evaluating the activity of paromomycin on *Leishmania* spp. were conducted in the 1960s (NEAL et al., 1968) for the species *L.* (*L.*) *tropica,* with promising results. In the 1980s, further *in vitro* and *in vivo* studies were conducted.

Clinical results conducted in the Old World include the efficacy of an ointment containing 15% paromomycin + 12% methyl-benzethonium chloride for *L.* (*L.*) *major* and *L.* (*L.*) *mexicana* infections (EL-ON et al., 1984; ALEXANDER et al., 1989). Methyl benzethonium was added to the formulation to act as a skin permeator and increase the penetration of paromomycin, but it causes an inflammatory reaction and discomfort, sometimes with enlargement of the ulcerated lesion.

In the New World, paromomycin (15%) and methyl benzethonium chloride (12%) ointment has been evaluated in Belize, where the most common species causing cutaneous leishmaniasis are *L. (V.) braziliensis* and *L.* (*L.*) *mexicana* (WEINRAUCH et al, 1993), in Ecuador (KRAUSE et al., 1994) and Guatemala (ARANA et al., 2001). The cure rate ranged from 72 to 86%, with prolonged treatment times, and the inflammatory skin reaction caused by methyl benzethonium was frequent.

The formulations mentioned above do not have good cutaneous permeation, requiring the addition of cutaneous permeators, which cause undesirable inflammatory reactions, sometimes with an increased lesion for increased efficacy.

More recently, the WR 279.396 formulation was developed by the Walter Reed National Military Medical Center, USA, and is being studied in phase II and phase III clinical trials in the Old World and Latin America. This is a cream for topical treatment of cutaneous leishmaniasis, containing 15% paromomycin and 0.5% gentamicin. In the "intention-to-treat" analysis of one of the studies, 94% of the Old World patients had a complete clinical cure. In other locations, the cure rate ranged from 87% (Panama) to 81% (Tunisia) (BEM SALAH et al., 2009; RAVIS et al., 2013; NESTOR et al., 2013; BEM SALAH et al., 2013).

The rationale for the association with gentamicin is for the treatment of bacterial infections, usually present in ulcerated lesions since gentamicin has no leishmanicidal activity.

### Other approaches

Topical drug application has been seen as an attractive alternative for the treatment of cutaneous leishmaniasis. Topical formulations offer significant advantages over systemic therapy, such as fewer adverse effects, ease of administration, and reduced cost. The different drugs studied for the topical treatment of CL, in addition to paromomycin, also include formulations of imiquimod, amphotericin B (AmB), miltefosine, and buparvaquone, all in development stages.

Most studies evaluating formulations containing antileishmanial drugs for topical treatment of cutaneous leishmaniasis have investigated conventional dosage forms such as ointments, creams, and gels. Although studies in experimentally infected animals have shown promising results, clinical trials have shown variable efficacy and sometimes disappointing results. This can be attributed, at least in part, to the low skin penetration of antileishmanial drugs. Therefore, new drug administration systems, such as lipid nanocarriers, have the potential to improve drug penetration into or through the skin.

Thus, one of the objectives of the present invention is to provide a fixed-dose topical composition containing at least one antileishmanial compound, providing adequate absorption of the active ingredient, and having sufficient efficacy so that it can be used in the effective treatment of cutaneous leishmaniasis.

Combining drugs can also help to increase efficacy, reduce the frequency of adverse effects, and prevent and/or delay the emergence of resistance. For the leishmaniases, experimental *in vitro* and *in vivo* studies conducted with species causing visceral leishmaniasis and cutaneous leishmaniasis have demonstrated the advantages of the combination of drugs, and this strategy is suggested for the clinical therapy of these diseases.

In Brazil, studies evaluating paromomycin combined with leishmanicidal drugs have shown promising results in animals. The first combination studies evaluated a 10% hydrophilic paromomycin gel applied twice daily for 10 days with oral miltefosine (10 mg/kg/day for 10 days) in animals infected with *L.* (*L.*) *amazonensis.* This combination significantly reduced the lesion size and parasite load in the skin and spleen of the animals (AGUIAR et al., 2010).

In another study by the same group, 10% paromomycin hydrophilic gel applied twice daily for 10 days was combined with oral miltefosine (25 mg/kg/day for 10 days) for the treatment of Balb/C mice experimentally infected with *L.* (*L.*) *major.* This drug combination reduced lesion size and local parasite load (lesion) compared to groups of animals receiving miltefosine alone or a placebo (AGUIAR et al., 2009).

Proposals for drug combinations for the treatment of cutaneous leishmaniasis are varied and seem to suggest superior efficacy when the combination treatment is compared to the reference drug alone (BAFICA et al, 2003; MACHADO et al., 2007; DASTGHEIB et al., 2012; SHANEHSAZ et al., 2015; FARAJZADEH et al., 2015; JAFFARY et al., 2016). In addition, by using lower doses of the drugs, combinations can reduce the frequency and severity of adverse effects, which can make them a relevant clinical alternative, especially for high-risk patients.

However, all the studies conducted so far for the treatment of cutaneous leishmaniasis are directed at the combined use of one drug applied topically, while another drug is applied systemically. Thus, even though a positive interaction between the drugs can be observed in some cases, a lot of the negative effects related to the systemic administration of the drugs are not mitigated.

Thus, another objective of the present invention is to provide a topical, fixed-dose formulation containing a combination of antileishmanial compounds that is capable of allowing adequate absorption of the compounds and that has sufficient efficacy and safety to be used in the treatment of cutaneous leishmaniasis.

### SUMMARY OF THE INVENTION

In a first aspect, the present invention relates to a composition comprising:
(a) a stable topical composition comprising a nanoemulsion, and
(b) at least one antileishmanial compound incorporated into said nanoemulsion.

In one approach, the antileishmanial compound is selected from meglumine antimoniate and sodium stibogluconate, amphotericin B, pentamidine isethionate, miltefosine, paromomycin, imiquimod, and buparvaquone, or combinations of these.

In another approach, the antileishmanial compound is selected from tamoxifen, meglumine antimonate, paromomycin, and amphotericin B, or combinations of these.

In a preferred approach, the antileishmanial compound is paromomycin.

In another preferred approach, the antileishmanial compound is a combination of paromomycin and amphotericin B.

In still another preferred approach, the antileishmanial compound is a combination of two or three selected from tamoxifen, meglumine antimoniate, and amphotericin B.

In another preferred approach, the antileishmanial compound is a combination of meglumine antimoniate and amphotericin B.

In one approach, according to the invention, the nanoemulsion comprises at least one non-ionic emulsifier, at least one amphoteric surfactant, at least one emollient, at least one humectant, and at least one moisturizer.

In another approach, at least one antileishmanial compound is incorporated into the oil globules of said nanoemulsion in the presence of one or more oxygen carriers and optionally one or more of this oily vehicle, permeation promoters, and moisturizers.

In another aspect, the present invention relates to a pharmaceutical composition comprising the composition according to the present invention, and at least one adjuvant and/or excipient.

In one approach, the pharmaceutical formulation is in the form of a suspension, emulsion, lotion, spray, unguent, cream, gel, poultice, film, ointment, or plaster.

In yet another aspect, the present invention relates to the use of a stable topical composition comprising a nanoemulsion and at least one antileishmanial compound for the manufacture of a medicament for the treatment of cutaneous leishmaniasis.

In a first approach, the antileishmanial compound is selected from meglumine antimoniate and sodium stibogluconate, amphotericin B, pentamidine isethionate, miltefosine, paromomycin, imiquimod, and buparvaquone, or combinations of these.

In another approach, the antileishmanial compound is selected from tamoxifen, meglumine antimonate, paromomycin, and amphotericin B, or combinations of these.

In a preferred approach, the antileishmanial compound is paromomycin.

In another preferred approach, the antileishmanial compound is a combination of paromomycin and amphotericin B.

In still another preferred approach, the antileishmanial compound is a combination of two or three selected from tamoxifen, meglumine antimoniate, and amphotericin B.

In another preferred approach, the antileishmanial compound is a combination of meglumine antimoniate and amphotericin B.

In another approach, the nanoemulsion comprises at least one non-ionic emulsifier, at least one amphoteric surfactant, at least one emollient, at least one humectant, and at least one moisturizer.

In yet another approach, at least one antileishmanial compound is incorporated into the oil globules of said nanoemulsion in the presence of one or more oxygen carriers and optionally one or more among oily vehicles, permeation promoters, and moisturizers.

In another aspect, the present invention relates to a method for treating cutaneous leishmaniasis that comprises administering a composition or a pharmaceutical composition according to the present invention to a patient in need thereof.

### BRIEF DESCRIPTION OF THE IMAGES

**Image 1** - Average lesion size (mm) of male hamsters (*Mesocricetus auratus*) (n=6) infected at the base of the tail with 200 µL suspension of *L.* (*V.*) *braziliensis* amastigotes and treated with spray nanoemulsions of paromomycin, amphotericin B, and combination of paromomycin + amphotericin. The lesions were measured every seven days, and the animals were euthanized thirty-three days after the start of treatment (D33). The vertical bars represent the standard deviation of the average of the largest diameter of the lesions in each group.

**Image 2** - Viable parasites in the lesion (A) and spleen (B) of male hamsters (*Mesocricetzis auratus*) (n=6) infected at the base of the tail with 200 µL suspension of *L.* (*V.*) *braziliensis* amastigotes and treated with spray nanoemulsions of paromomycin, amphotericin B and combination of paromomycin + amphotericin. The parasites were determined using the limiting dilution technique on the day the animals were euthanized, D33 (33 days from the start of treatment). The horizontal bars represent the average number of viable parasites in the lesions or spleens of each group. * There was bacterial contamination on the lesion parasite culture plates from three animals in the PA 10% + AnfB 3% group.

**Image 3** - Average size of the largest diameter of lesions (mm) of male hamsters (Mesocricetus auratus) (n = 4) infected at the base of the tail with 1x105/200µL metacyclic promastigotes of L. (V. ) braziliensis and treated with combinations of spray nanoemulsions of amphotericin B 6% + meglumine antimoniate 12%, amphotericin B 6% + tamoxifen 0.5%; amphotericin B 6% + meglumine antimoniate 12% + tamoxifen 0.5% and the isolated formulations amphotericin B 6%, meglumine antimoniate 12% and tamoxifen 0.5%.

### DETAILED DESCRIPTION OF THE INVENTION

Unless defined differently, all technical and scientific terms used herein have the same meaning as understood by someone with expertise in the subject matter to which the invention pertains. Conventional molecular biology and immunology techniques are well known to an expert in the field. The narrative report also provides definitions of terms to assist in the interpretation of what is described here and the claims. Unless otherwise indicated, all figures expressing quantities, percentages, proportions, and other numerical values used in the descriptive report and in claims are to be understood as being modified in all cases by the term "about". Thus, unless otherwise stated, the numerical parameters shown in the descriptive report and in the claims are approximations that may vary depending on the properties to be obtained.

The present invention comprises a topical composition containing at least one antileishmanial compound.

Among the antileishmanial compounds according to the present invention, we can highlight pentavalent antimonial compounds, such as meglumine antimonate and sodium stibogluconate, amphotericin B, pentamidine isethionate, miltefosine, paromomycin, imiquimod, and buparvaquone. Preferably, the present invention is about a combined fixed-dose topical formulation of paromomycin and amphotericin B.

According to the present invention, the antileishmanial compounds are incorporated into the formulation through carriers, such as microcarriers and nanocarriers.

When applied to intact skin, microcarriers and nanocarriers can increase dermal or transdermal penetration of drugs, depending on the composition and size of the vesicles. Different mechanisms of action for micro and nanocarriers as drug administration systems into the skin have been suggested: enhanced penetration by the individual particle components; adsorption of the vesicle and/or merging with the stratum corneum (SC); intact penetration of the particle into and through intact skin; and follicular penetration.

The use of micro and nanocarriers in the topical treatment of cutaneous leishmaniasis, when compared to conventional formulations, may be beneficial because, when such particles are applied to skin with a normal or compromised barrier, they can dramatically increase drug penetration.

Among the preferred micro and nanocarriers, according to the present invention, we can highlight microemulsions and nanoemulsions.

According to the present invention, microemulsions are aqueous dispersions of particles, averaging in size between 1 nm and 1,000 µm, composed of a lipid core surrounded by monolayers of surfactants and/or co-surfactants. Nanoemulsions, which are a subgroup of microemulsions, are colloidal systems that include micelles, liposomes, virosomes, nanosuspensions, and other polymeric solutions, with an average size between 1 nm and 10,000 nm.

Preferably, the antileishmanial drugs according to the present invention shall be incorporated into the nanoemulsion, hereinafter referred to as Biolipid B2. Biolipid B2, developed by Evidence Group, was first described in Brazilian patent PI 1002486-7, as well as its preparation process and methods for incorporating active ingredients in the composition described. Biolipid B2 is a stable, biocompatible nanoemulsion capable of delivering drugs for transdermal administration.

According to the present invention, when antileishmanial drugs are incorporated into Biolipid B2, the latter can be used directly as a final product or additionally mixed with other adjuvants and excipients to form other compositions suitable for topical administration.

Some examples of these other formulations suitable for topical administration include suspensions, emulsions, lotion sprays, unguents, creams, gels, plasters, films, ointments, and adhesive-incorporated compositions, all of which are known in the technique of topical formulations and preparations. Lotion, cream, ointment, and spray formulations are preferred according to the present invention.

Among the adjuvants and excipients that can be used for the preparation of nanoemulsions, Biolipid B2, or the compositions according to the present invention, can be highlighted:
the permeation enhancers, such as dibutyl adipate, isopropyl myristate, dimethyl sulfoxide, diethylene glycol monoethyl ether, propylene glycol dicaprylocaprate, isopropyl myristate, sodium lauryl sulfate, polyoxyethylene sorbitan monooleate, and sorbitan monolaurate;
the oxygen carriers selected from perfluorocarbons, preferably perfluorotrialkylamines, such as perfluorohexane, perfluorodimethylcyclohexane, octofluorooctane, and perfluorodecalin;
fatty alcohols such as those having about 4 to 30 carbon atoms, such as stearyl alcohol, cetyl alcohol, cetostearyl alcohol, and myristyl alcohol;
emollients such as dibutyl adipate, diisobutyl adipate, diisopropyl adipate, dimethicone, fatty acid triglyceride esters such as caprylic/capric triglycerides, hydroxylated lanolin, isopropyl myristate, mineral oil, soy sterol, cetyl stearate and petrolatum, linolenic acid, linoleic acid and oleic acid;
emulsifiers, such as steareth-2, steareth-21, glyceryl monostearate SE (a mixture of glyceryl stearate and PEG-100 stearate) and laureth-4, cetearyl, sorbitan, and ceteareth from mixtures of fatty acid esters resulting from the saponification of vegetable oil, selected from coconut oil, palm oil, olive oil, soybean oil, sunflower seed oil, or animal oil;
humectants such as glycerin, propylene glycol, sorbitol, lactose, mannitol, sodium pyrrolidone carboxylic acid, panthenol, hyaluronic acid, and chondroitin;
the amphoteric surfactants such as saponins, lecithin, and soy proteins; and
the moisturizers such as trehalose, maltose, and sucrose;
as well as any combinations or mixtures thereof, and other similar and equivalent compounds.

Other additives may also be incorporated into the compositions of the present invention, such as ultraviolet absorbers or sunscreens, antioxidants, preservatives, and others, for improved stability during use and storage. Non-limiting examples of appropriate antioxidants and preservatives include, but are not limited to, butylated hydroxytoluene, butylated hydroxyanisole (BHA), sorbic acid, benzoic acid, benzyl alcohol, imidazolidinyl urea, diazolidinyl urea, methylparaben, propylparaben, potassium sorbate, and mixtures or combinations thereof.

It should also be understood that the compositions of the present invention may include other components commonly used in conventional topical cosmetic formulations, such as suspending agents, thickening agents, film formers, preservatives, and fragrance oil. The thickening agents are preferably those that are compatible with the composition, such as bentones, xanthan gum, silica, and ethyl cellulose. Dyes, fragrances, and other cosmetic additives may also be present. The exemplified and specifically listed cosmetic components may be freely substituted with other conventional and well-known components to obtain the desired texture and lubricity of the compositions, on the condition that the substitutes do not react adversely with any component of the composition and do not interfere with the homogeneity of the composition.

The present invention is also described by the non-limiting example below, which is merely illustrative. Various modifications and variations of the embodiments are evident to the expert in the subject without straying from the spirit and scope of the invention.

Numerous variations affecting the scope of protection of the present application are allowed. Thus, it is reinforced that the present invention is not limited to the particular configurations / embodiments described above.

### EXAMPLES

### EXAMPLE 1

### Objective

Evaluate nanoemulsions in spray presentation of paromomycin, amphotericin B, and the combination of paromomycin + amphotericin for the treatment of skin lesions caused by *Leishmania (Viannia) braziliensis* in experimentally infected hamsters. This model is on the spectrum of susceptibility to infection, difficult therapeutic responses, and a tendency to reactivation with parasite persistence, even when leishmanicides with good action in humans are used.

### Development

### PARASITES

The study was conducted with the reference strain *Leishmania (V.) braziliensis* MHOM/BR/75/M2903, and with the reference strain *Leishmania (Leishmania) major* (MHOM/IL/80/Friendlin), characterized and deposited in the strain bank of the *Leishmania* Collection of the Reference Center for *Leishmania* Typing at the Instituto Oswaldo Cruz.

### ANIMALS

The animals used in this study were kept in the vivarium of the Instituto René Rachou (IRR) and the procedures performed were previously approved by the Ethics Committee on Animal Use of the Fundação Oswaldo Cruz - Permit LW06/13.

### FORMULATIONS EVALUATED

Samples containing antileishmanial drugs incorporated into Biolipid B2 nanoemulsions, produced according to the preparation process described in patent PI 1002486-7.

| **Samples** |
|---|
| Paromomycin Sulfate 10% P/V + Amphotericin B 3% P/V - PH: 6.7 |
| Paromomycin Sulfate 10% P/V - PH: 6.7 |
| Amphotericin B 6% P/V - PH: 6.7 |

### INFECTION AND TREATMENT OF THE ANIMALS - L. (V.) BRAZILIENSIS

Twenty-eight male hamsters *(Mesocricetus auratus*) of approximately 148.7g of body mass were infected by subcutaneous injection at the base of the tail with 200µL of a suspension of amastigotes of the *L. (V.) braziliensis* M2903 strain. After 52 days of evolution (04/11/18 - start of treatment), the average size (largest diameter) of the skin lesions was measured with a digital caliper (12.5 mm ± 2.5 mm). For each treatment, the animals were grouped into seven groups of four animals each. Grouping was performed in such a way that the average lesion sizes between the groups were similar.

### TREATMENT GROUPS:

**1 - PA 10% + AnfB 3%:** Paromomycin Sulfate 10% P/V + Amphotericin B 3% P/V - pH: 6.7, administered twice a day, for 30 consecutive days;
**2 - AnfB 6%:** Amphorericin B 6% P/V - pH: 6.7, administered twice a day, for 30 consecutive days;
**3 - PA 10%:** Paromomycin Sulfate 10% P/V - pH: 6.7, administered twice a day, for 30 consecutive days;
**4 - Control:** No treatment control: infected and untreated animals.

### Evaluation of clinical efficacy

The effectiveness of the treatment was evaluated by weekly measurement of the size of the lesions. Before, during, and after treatment, the average lesion size (mm) was determined by measuring the largest lesion diameter using a digital caliper. Measurement was performed at D1 - the day treatment started, D7 - 7 days after treatment started, D14 - 14 days after treatment started, D21 - 21 days after treatment started, D28 - 28 days after treatment started, and D33 - 3 days after treatment ended (the day the animals were euthanized). The percentage reduction in average lesion size calculated by the difference in lesion size at the start of treatment (D1) and three days after the end of treatment (D33) was determined. The percentage of animals that had complete healing of the lesion was determined at D33.

### EVALUATION OF PARASITOLOGICAL EFFICACY

To evaluate the effect of treatment in reducing or eliminating the parasite load of infected and treated animals, three days after the end of treatment (D33), the lesion and spleen were removed, ground in a tissue homogenizer, centrifuged, and the contents of the final pellet were distributed on culture plates in a 10x serial dilution. After seven days of culture, the plate wells were read under an inverted microscope and viable parasites were determined.

### EVALUATION OF TREATMENT TOXICITY

Every seven days (D1, D7, D14, D21, D28, and D33 - three days after the end of treatment), the animals were weighed to evaluate the possible toxic effects of the treatment. General physical aspects such as piloerection, behavioral changes, diarrhea, and others were also observed for this purpose.

### DATA ANALYSIS

The data were processed using GraphPad Prism 5 software. To compare parasite loads, animal weights, and lesion sizes between groups, an one-way analysis of variance followed by Tukey's test was applied. The difference was considered significant when the p-value was less than 0.05. Parasite load data were log10 +1 transformed and evaluated for normality using the Kolmogorov-Smirnov test.

### Results

**Clinical Efficacy:** The reduction in lesion size over time for each treatment regimen was evaluated. A statistically significant difference (*p<0.05) compared to the untreated control group was observed for the group of animals treated with the combination PA 10% + AnfB 3%, starting on D21 (21 days after the start of treatment). For the other groups, despite the evidence of reduction in the average size of the lesions, there was no statistically significant difference at any of the times evaluated (p > 0.05) (IMAGE 1).

The percentage of lesion size reduction was calculated on the day of euthanasia (D33) compared to the initial treatment time (D1) and the percentage of complete healing of the animals (TABLE 1). A 94.2% reduction in lesion size is observed for the PA 10%+AnfB 3% group. This group also showed 83.3% (5/6) complete healing of the lesion. The AnfB 6% and PA 10% groups showed a reduction percentage of 16.6% and 50%, respectively. Lesion healing of the animals in the 10% PA group was 50%, and there was no complete healing for the animals in the 6% AnfB group. There was no reduction in the size of the animals' lesions and complete healing of the lesion for the untreated control group (TABLE 1).

**TABLE 1 - Clinical efficacy of paromomycin, amphotericin B, and paromomycin + amphotericin B combination spray nanoemulsions in hamsters experimentally infected with L. (V.) braziliensis**

| **Group** | **Clinical Efficacy** | |
|---|---|---|
| | **% reduction in lesion size¹** | **% of animals with complete healing of the lesion²** |
| PA10%+AnfB 3% | 94.2 | 83.3 (5/6) |
| AnfB 6% | 16.6 | 0.0 (0/6) |
| PA 10% | 50.0 | 50.0 (3/6) |
| Control | 0.0 | 0.0 (0/6) |

| | | |
|---|---|---|
| * The average size of lesions was calculated by the largest diameter (mm). The percentages of average lesion size reduction and complete healing were obtained by considering the ratio between the average lesion values at time D1 (start of treatment) and D33 (day of euthanasia). | | |

### PARASITOLOGICAL EFFICACY

Regarding the evaluation of the parasite load, there was a statistically significant difference in the lesion (A) for the group of animals treated with the combination of paromomycin + amphotericin (PA 10% + AnfB 3%) compared to the untreated control. In the groups that received paromomycin (PA 10%) and amphotericin B (AnfB 6%) alone, no statistically significant difference was observed. In the spleen, a statistically significant difference was observed for the 6% AnfB group compared to the untreated control (*p<0.05) (IMAGE 2).

Drug toxicity was evaluated through body mass during and after treatment. The animals did not lose weight at any time during the evaluation, nor did they show signs that could indicate toxicity (raised fur, aggressiveness, diarrhea, or others).

### CONCLUSIONS

Paromomycin 10% spray nanoemulsion showed moderate efficacy in *L. (V.) braziliensis* infection leading to reduction of lesion size by 50%. Amphotericin B 6% nanoemulsion showed moderate efficacy in reducing the average size and *viable parasites in the lesion* and was also able to significantly reduce the viable parasite load in the spleen of the animals, a finding not observed with PA 10% and well with the combination of PA 10% and AnfB 3%.

The combination nanoemulsion of Paromomycin 10% + Amphorericin B 3% was significantly effective in reducing the average lesion size and viable parasite load in the animals' lesions.

### EXAMPLE 2

### Objective

To evaluate spray-presentation nanoemulsions of amphotericin B, meglumine antimonate, and tamoxifen and combinations of amphotericin B + meglumine antimoniate, amphotericin B + tamoxifen, and amphotericin B + meglumine antimoniate + tamoxifen for the treatment of skin lesions caused by *Leishmania (Viannia) braziliensis* in experimentally infected hamsters.

### Development

### PARASITES

The study was conducted with the reference strain *Leishmania (V.) braziliensis* MHOM/BR/75/M2903.

### ANIMALS

In this study, the experimental infection model was used in golden hamsters*(Mesocricetus auratus).* The animals used were kept in the vivarium of the Instituto René Rachou (IRR) and the procedures performed were previously approved by the Ethics Committee on Animal Use of the Fundação Oswaldo Cruz - Permit LW04/20.

### EVALUATED FORMULATIONS

Samples containing antileishmanial drugs incorporated into Biolipid B2 nanoemulsions, produced according to the preparation process described in patent PI 1002486-7.

| **Samples** |
|---|
| Formula 1-Amphotericin B 6% + Meglumine antimoniate 12% Extremely absorbable biolipid |
| Formula 2-Amphotericin B 6% + Tamoxifen 0.5% Extremely absorbable biolipid |
| Formula 3-Amphotericin B 6% + Meglumine antimoniate 12% + tamoxifen 0.5% Extremely absorbable biolipid |
| Formula 4-Amphotericin B 6%. Extremely absorbable biolipid |
| Formula 5-Meglumine antimoniate 12%. Extremely absorbable biolipid |
| Formula 6-Tamoxifen 0.5%. Extremely absorbable biolipid |

### INFECTION AND TREATMENT OF THE ANIMALS - L. (V.) BRAZILIENSIS

Twenty-eight male hamsters*(Mesocricetus auratus*) of approximately 135.5g of body mass were infected by subcutaneous injection at the base of the tail with 1x10⁵/200µL metacyclic promastigotes of the *L*. *(V.) braziliensis* M2903 strain. After 50 days of evolution (9/16/21 - start of treatment), the average size (largest diameter) of the skin lesions was measured with a digital caliper (13.3 mm ± 2.9 mm). For each treatment, the animals were grouped into seven groups of five or four animals each. Grouping was performed in such a way that the average lesion sizes between the groups were similar.

### TREATMENT GROUPS:

**F1** - Amphotericin B 6% + Meglumine antimoniate 12%, administered twice a day, topically (spray) for 49 consecutive days;
**F2** - Amphotericin B 6% + Tamoxifen 0.5%, administered twice a day, topically (spray) for 49 consecutive days;
**F3** - Amphotericin B 6% + Meglumine antimoniate 12% + tamoxifen 0.5%, administered twice a day, topically (spray) for 49 consecutive days;
**F4** - Amphotericin B 6%, administered twice a day, topically (spray) for 49 consecutive days;
**F5-** Meglumine antimoniate 12%, administered twice a day, topically (spray) for 49 consecutive days;
**F6-** Tamoxifen 0.5%, administered twice a day, topically (spray) for 49 consecutive days;
**F7-** Control: infected and untreated animals.

### EVALUATION OF CLINICAL EFFICACY

The effectiveness of the treatment was evaluated by weekly measurement of the size of the lesions using a digital caliper (Digimess). Before, during, and after treatment, the average lesion size (mm) was determined by measuring the largest lesion diameter. Measurement was performed at D1 - the day treatment started, D7 - 7 days after treatment started, D14 - 14 days after treatment started, D21 - 21 days after treatment started, and D28 - 28 days after treatment started, D35 - 35 days after treatment started, D42 - forty-two days after treatment started, and D49 - forty-nine days after treatment started. The percentage reduction in average lesion size was calculated by the difference in lesion size at the start of treatment (D1) and 49 days after the start of treatment (D49). The percentage of animals that showed complete healing of the lesion was determined at D49. The magnitude of the change in lesion size between the start of treatment and the end of observation was also determined by the ratio of the largest ulcer diameter at D49 to the largest lesion diameter on the first day of treatment (D1), as well as the proportion of animals with complete epithelialization at D49.

### EVALUATION OF TREATMENT TOXICITY

Every seven days (D1, D7, D14, D21, D28, D35, D42, and D49), the animals were weighed to evaluate the possible toxic effects of the treatment. General physical aspects such as pilo-erection, behavioral changes, diarrhea, and others were also observed for this purpose.

### DATA ANALYSIS

The database for this study was built using Microsoft Office Excel 2007 spreadsheets, exported to GraphPad Prism 5 for Windows (GraphPad Software, San Diego, California, USA).

The analysis strategy consisted of comparing the groups with the different treatments in relation to the parameter of clinical evolution of the animal's skin lesion. When evaluating the clinical response, in addition to the graph of the evolution of lesion measurements (largest ulcer diameter), the magnitude of ulcer reduction, calculated by the ratio of the largest lesion diameter on D49 in relation to the largest lesion diameter on D0 (start of treatment), and the number of animals in each group that showed complete epithelialization of the ulcer were also evaluated.

Comparisons were performed by parametric and nonparametric hypothesis tests, and the distribution of the continuous variables (weight and lesion size) was assessed for normality by the Kolmogov-Sminrnov (KS) and D'Agostino & Pearson tests. For continuous variables with normal distribution, comparison of averages was done using Tukey's multiple comparisons test for comparison of more than two groups. Analysis of continuous variables with non-normal distributions was performed by comparing averages using the Kruskal-Wallis test for three or more groups. For all comparisons, the significance level considered was 5%.

### RESULTS

At D1 and D7, the groups showed no statistically significant difference between them (p>0.5). At D14, the group amphotericin B 6% + meglumine antimoniate 12% + tamoxifen 0.5% showed a statistically significant difference compared to the untreated control, tamoxifen 0.5% group, and amphotericin B 6% (p<0.05).

At D21, the group amphotericin B 6% + meglumine antimoniate 12% started to show a statistically significant difference compared to the untreated control and tamoxifen 0.5% group (p<0.05).

At D28, significant statistical differences were observed between the amphotericin B 6% + meglumine antimoniate 12% groups compared to the control group and tamoxifen 0.5% (p<0.05). At the same time, the group amphotericin B 6% + meglumine antimoniate 12% + tamoxifen 0.5% showed a statistically significant difference compared to the animals treated with tamoxifen 0.5% (p<0.5).

At D35, the statistical differences observed at D28 for the amphotericin B 6% + meglumine antimoniate 12% group remain , and also between this group and the amphotericin B 6% treatment (p<0.5). The combined formulation of amphotericin B 6% + meglumine antimoniate 12% + tamoxifen 0.5% showed a statistically significant difference at D35 compared to the tamoxifen 0.5% treated group (p<0.5).

At D42 and D49, the statistically significant differences observed at D35 for the group of animals treated with the combination amphotericin B 6% + meglumine antimoniate 12% remain (p<0.5) and at D49, the triple combination of amphotericin B 6% + meglumine antimoniate 12% + tamoxifen 0.5% showed a statistically significant difference compared to the tamoxifen 0.5% group and also the untreated control group (P<0.5).

Lesions were measured every seven days at the beginning (1), seven (7), 14, 21, 28, 35, 42, and 49 days after treatment. The vertical bars in IMAGE 3 represent the standard deviation of the average of the largest diameter of the lesions in each group. At D49, a statistically significant difference (p<0.05) was observed for the group of animals treated with the combined formulation of meglumine antimoniate 12% and amphotericin B 6% compared to the untreated control and the isolated formulations of tamoxifen 0.5% and amphotericin B 6% and between the group amphotericin B 6% + meglumine antimoniate 12% + tamoxifen 0.5% and the untreated control and tamoxifen 0.5% (P<0.05).

The percentage reduction in lesion size was calculated from the measurements taken on day 49 (D49) of treatment compared to the measurements taken on the first day of treatment (D1) (TABLE 2). At D49, a reduction in lesion size of 65.9% is observed for the amphotericin B + meglumine antimoniate group; 52.6% for the amphotericin B + meglumine antimoniate + tamoxifen group; and 25.3% for the group treated with meglumine antimoniate alone. Complete lesion healing for these groups was 40%, 20%, and 0%, respectively. There was no reduction in the size of the animals' lesions and complete healing of the lesion for the untreated control group and the other treatment groups (TABLE 2).

The calculation of lesion magnitude (TABLE 2) confirmed the reduction in lesion size for treatments with the formulas amphotericin B 6% + meglumine antimoniate 12%; amphotericin B 6% + meglumine antimoniate 12%, + tamoxifen 0.5% and meglumine antimoniate 12%.

**TABLE 2 - Clinical efficacy of spray nanoemulsions of leishmanicidal drugs amphotericin, meglumine antimoniate, and tamoxifen combined and isolated produced by Evidence in hamsters experimentally infected with L. (V.) braziliensis.**

| **Group** | **Clinical Efficacy** | | **Magnitude of lesion variation: D49/D1* (average±SD)** |
|---|---|---|---|
| | **% reduction in lesion size¹** | **% of animals with complete complete lesion** | |
| AnfB 6% + AM 12% | 65.9 | 40.0 (2/5) | 0.40±0.5 |
| AnfB 6% + Tamoxi 0.5% | 0.0 | 0.0 (0/5) | 1.22±0.3 |
| AnfB 6% + AM 12% + Tamoxi 0.5% | 52.6 | 20.0 (1/5) | 0.46±0.3 |
| AnfB 6% | 0.0 | 0.0 (0/5) | 1.13±0.3 |
| AM 12% | 25.3 | 0.0 (0/5) | 0.75±0.2 |
| Tamoxifen 0.5%. | 0.0 | 0.0 (0/4) | 0.98±0.1 |
| Control | 0.0 | 0.0 (0/4) | 1.04±0.1 |

| | | | |
|---|---|---|---|
| The average size of the lesions was calculated by the largest diameter (mm). ¹ The percentages of reduction in the average size of the lesions were obtained by considering the ratio between the average values of the lesions at D1 (the start of treatment) and D49 (49 days after the start of treatment). *calculated as the ratio of the average of the largest lesion diameter at D49 (49 days after the start of treatment) to the average of the largest lesion diameter at D1 (the start of treatment). AnfB 6% = Amphotericin B 6%; AM 12% = meglumine antimoniate 12% and Tamoxi 0.5% = tamoxifen 0.5%. | | | |

### FINAL CONSIDERATIONS:

Drug toxicity was evaluated by measuring body mass during and after treatment. The animals did not lose weight at any time during the evaluation, nor did they show signs that could indicate toxicity (raised fur, aggressiveness, diarrhea, or others). No animals died by the end of the experiment (D49).

The nanoemulsions containing the leishmanicidal drugs: a) amphotericin B 6% + meglumine antimoniate 12%; and b) amphotericin B 6% + meglumine antimoniate 12% + tamoxifen 0.5%, were effective in reducing the size of experimentally induced cutaneous leishmaniasis lesions in an animal model, compared to the control group of untreated animals;

### FINAL CONCLUSION

The nanoemulsion containing the leishmanicidal drugs amphotericin B 6% + meglumine antimoniate 12% is sufficient and shows superior therapeutic capacity than that obtained with the drugs alone, as demonstrated by the significant reduction in the size of experimentally induced cutaneous leishmaniasis lesions in an animal model, compared to the untreated control group.

### REFERENCES

ACHTERBERG, V.; GERCKEN, G. Metabolism of ether lysophospholipids in Leishmania donovani promastigotes. Mol. Biochem. Parasitol., v. 26, n. 3, p. 277-287, 1987.
AGUIAR, M. G. et al. Combined topical paromomycin and oral miltefosine treatment of mice experimentally infected with Leishmania (Leishmania) major leads to reduction in both lesion size and systemic parasite burdens. J. Antimicrob. Chemother., v. 64, n. 6, p. 1234-1240, 2009.
AGUIAR, M. G. et al. Reductions in skin and systemic parasite burdens as a combined effect of topical paromomycin and oral miltefosine treatment of mice experimentally infected with Leishmania, (Leishmania) amazonensis. Antimicrob. Agents Chemother., v. 54, n. 11, p. 4699-4704, 2010.
BALANA-FOUCE, R. et al. The pharmacology of leishmaniasis. Gen. Pharmacol., v. 30, n. 4, p. 435-443, 1998.
BERMAN, J. Chemotherapy for leishmaniasis: biochemical mechanisms, clinical efficacy, and future strategies. Rev. Infect. Dis., v. 10, n. 3, p. 560-586, 1988.
BERMAN, J. Clinical status of agents being developed for leishmaniasis. Expert. Opin. Investigate. Drugs, v. 14, n. 11, p. 1337-1346, 2005.
BRASIL. Ministério da Saúde. Secretaria de Ciência, Tecnologia e Insumos Estratégicos Relatório de Recomendação: Miltefosina para o tratamento de leishmaniose tegumentar. Brasilia: Ministério da Saúde, 2016.
BRASIL. ANVISA- Instrução Normativa Anvisa N° 2, de 30 de Março de 2009. Guia para Notificação de Lotes-Piloto de Medicamentos, 2016.
BRASIL. Ministério da Saúde. Secretaria de Vigilância em Saúde, Departamento de Vigilância das Doenças Transmissíveis. Manual de Vigilância da Leishmaniose Tegumentar Americana. Brasilia: Editora do Ministério da Saúde 2017a.
BRASIL. DATASUS. Available at: < http://datasus.saude.gov.br >.
CARNEIRO, G. et al. Drug delivery systems for the topical treatment of cutaneous leishmaniasis. Expert Opin. Drug Deliv., v. 9, n. 9, p. 1083-1097, 2012.
CROFT, S. L. et al. The activity of alkyl phosphorylcholines and related derivatives against Leishmania donovani. Biochem. Pharmacol., v. 36, n. 16, p. 2633-2636, .1987.
DASTGHEIB, L.; NASERI, M.; MIRASHE, Z. Both combined oral azithromycin plus allopurinol and intramuscular Glucantime yield low efficacy in the treatment of Old World cutaneous leishmaniasis: a randomized controlled clinical trial. Int. J. Dermatol., v. 51, n. 12, p. 1508-1511, 2012.
ESCOBAR, P. et al. Sensitivities of Leishmania species to hexadecylphosphocholine (miltefosine), ET-18-OCH (3) (edelfosine) and amphotericin B. Acta Trop., v. 81, n. 2, p. 151-157, 2002.
FARAJZADEH, S. et al. Comparison between Combination Therapy of Oral Terbinafine and Cryotherapy versus Systemic Meglumine Antimoniate and Cryotherapy in Cutaneous Leishmaniasis: A Randomized Clinical Trial. Iran. J. Parasitol., v. 10, n. 1, p. 1-8, 2015.
HERRMANN, H. O.; GERCKEN, G. Metabolism of 1-0-[1'-14C]octadecyl-sn-glycerol in Leishmania donovani promastigotes. Ether lipid synthesis and degradation of the ether bond. Mol. Biochem. Parasitol., v. 5, n. 2, p. 65-76, 1982.
JAFFARY, F. et al. A Comparison between the Effects of Glucantime, Topical Trichloroacetic Acid 50% plus Glucantime, and Fractional Carbon Dioxide Laser plus Glucantime on Cutaneous Leishmaniasis Lesions. Dermatol. Res. Pract., v. 2016, 2016.
MACHADO, P. R. et al. Oral pentoxifylline combined with pentavalent antimony: a randomized trial for mucosal leishmaniasis. Clin. Infect. Dis., v. 44, n. 6, p. 788-793, 2007.
MOHEBALI, M. et al. Comparison of miltefosine and meglumine antimoniate for the treatment of zoonotic cutaneous leishmaniasis (ZCL) by a randomized clinical trial in Iran. Acta Trop. v. 103, n. 1, p. 33-40, 2007.
NEAL, R. A. The effect of antibiotics of the neomycin group on experimental cutaneous leishmaniasis. Ann. Trop. Med. Parasitol., v.62, n.1, p. 54-62, 1968.
ROBERT, A.; BRIGGAMAN, M. D. The aromati diamidines. Int. J. Dermatol., v. 16, n. 3, p. 155-162, 1977.
SAHA, A. K.; MUKHERJEE, J.; BRADIERE, A. Mechanism of action of amphotericin B on Leishmania donovani promastigotes. Mol. Biochen. Parasitol., v. 19, p. 195-200, 1986.
SHANEHSAZ, S. M.; ISHKHANIAN, S. A comparative study between the efficacy of oral cimetidine and low-dose systemic meglumine antimoniate (MA) with a standard dose of systemic MA in the treatment of cutaneous leishmaniasis. Int. J. Dermatol., v. 54, n. 7, p. 834-838, 2015.
SUNDAR, S.; CHAKRAVARTY, J. Paromomycin in the treatment of leishmaniasis. Expert Opin. Investigate. Drugs, v. 17, n. 5, 2008.
WHO. Leishmaniasis Epidemiological Situation. 2018. Available at: < http://www.who.int/leishmaniasis/burden/en/ >.

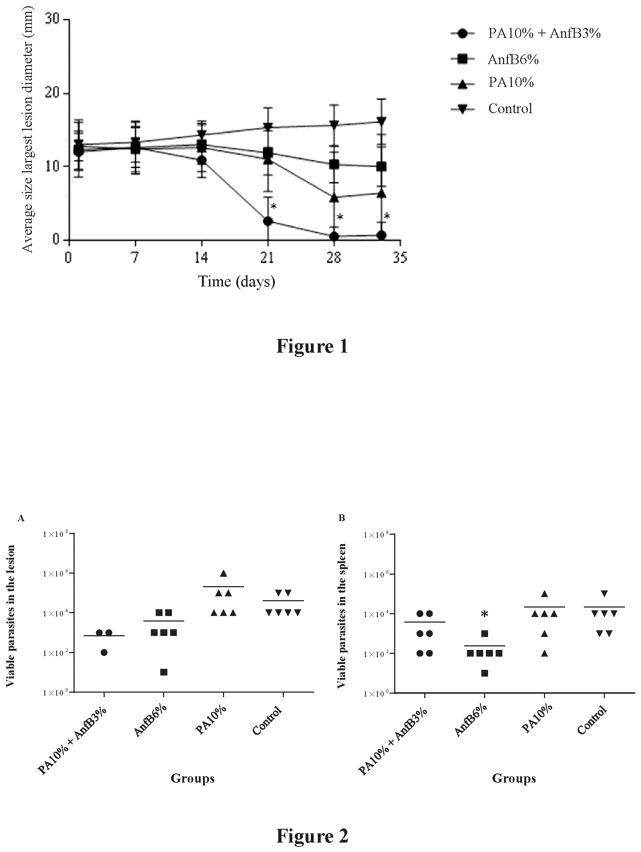

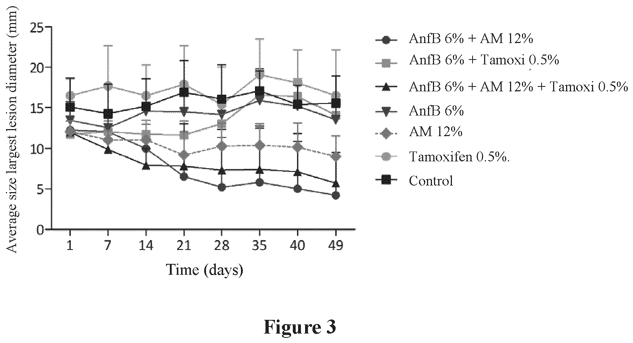

## Claims

1. A composition **characterized by** the fact that it comprises:
(a) a stable topical composition comprising a nanoemulsion, and
(b) a combination of the antileishmanial compounds meglumine antimoniate, and amphotericin B incorporated into said nanoemulsion.

2. A composition according to claim 1, **characterized by** the fact that the nanoemulsion comprises at least one non-ionic emulsifier, at least one amphoteric surfactant, at least one emollient, at least one humectant, and at least one moisturizer.

3. A composition according to claim 1 or 2, **characterized by** the fact that the antileishmanial compounds are incorporated into the oil globules of said nanoemulsion in the presence of one or more oxygen carriers and optionally one or more oily vehicles, permeation promoters, and moisturizers.

4. A pharmaceutical composition **characterized by** the fact that it comprises a composition, as defined in any one of the claims 1 to 3, and at least one adjuvant and/or excipient.

5. A pharmaceutical composition according to claim 4, **characterized by** the fact that it is in the form of a suspension, emulsion, lotion, spray, unguent, cream, gel, plaster, film, ointment, or adhesive.

6. The composition according to any of claims 1 to 3 or the pharmaceutical composition according to claim 4 or 5 **characterized by** the fact that it is for use in the treatment of cutaneous leishmaniasis.

7. The composition or the pharmaceutical composition for use, according to claim 6, **characterized by** the fact that the nanoemulsion comprises at least one non-ionic emulsifier, at least one amphoteric surfactant, at least one emollient, at least one humectant, and at least one moisturizer.

8. The composition or the pharmaceutical composition for use, according to claim 6 and 7, **characterized by** the fact that the antileishmanial compounds are incorporated into the oil globules of said nanoemulsion in the presence of one or more oxygen carriers and optionally one or more oily vehicle, permeation promoters, and moisturizers.
